# EUROPEAN PATENT APPLICATION

(11) **EP 3 485 760 A1**
(43) Date of publication of application: **22.05.2019**
(21) Application number: 17202083.6
(22) Date of filing: 16.11.2017
(51) Int. Cl.: A45D 29/02, A61B 5/00, A45D 44/00

(54) **NAIL CLIPPER**

(71) Applicant: Nokia Technologies Oy, 02610 Espoo (FI)
(72) Inventor: KIVIOJA, Jani, 02680 Espoo (FI); PELLIKKA, Jarkko, 00220 Helsinki (FI)
(74) Representative: Espatent Oy

(57) **Abstract**

An apparatus including nail clipping edges (103, 104) configured to move towards each other when a force is applied to the apparatus, and one or more strain gauges (120, 121, 122) configured to sense transformations of the apparatus caused by said force.

## Description

### TECHNICAL FIELD

The present application generally relates to nail clipping.

### BACKGROUND

This section illustrates useful background information without admission of any technique described herein representative of the state of the art.

Measuring or monitoring personal health conditions often require analysis of a blood sample. For example personal calcium or vitamin D levels are commonly analysed from a blood sample. Vitamin D adequacy may be determined by measurement of the 25-hydroxyvitamin D concentration in the blood sample.

Also characteristics of human or animal nails or changes thereof may be used as an indicator of certain health conditions. Characteristics of nails may be usable for example as an indicator of personal calcium or vitamin D levels.

### SUMMARY

Various aspects of examples of the invention are set out in the claims.

According to a first example aspect of the present invention, there is provided an apparatus comprising:
nail clipping edges configured to move towards each other when a force is applied to the apparatus, and
one or more strain gauges configured to sense transformations of the apparatus caused by said force.

In an example embodiment, the one or more strain gauges comprise a first strain gauge configured to sense a transformation that is proportional to displacement of the nail clipping edges.

In an example embodiment, the one or more strain gauges comprise a second strain gauge configured to sense a transformation that is proportional to the force applied to the apparatus.

In an example embodiment, the one or more strain gauges comprise a third strain gauge configured to sense transformation that is proportional to length of free edge of a nail placed between the nail clipping edges.

In an example embodiment, the apparatus further comprises a guiding member configured to guide a nail between the nail clipping edges.

In an example embodiment, the apparatus further comprises a memory configured to store readings of the one or more strain gauges.

In an example embodiment, the apparatus further comprises a communication interface configured to transmit readings of the one or more strain gauges to an external device.

In an example embodiment, the apparatus further comprises a processor configured to obtain a set of readings from the one or more strain gauges and to combine the set of readings into one reading.

In an example embodiment, the apparatus further comprises a processor configured to associate readings of the one or more strain gauges with a time stamp.

In an example embodiment, the one or more strain gauges comprise: a metal thin film based strain gauge, a nanoparticle based strain gauge, an elastomer strain gauge, a piezoelectric polymer strain gauge, a semiconductor strain gauge, or a fiber-optic strain gauge.

In an example embodiment the apparatus is a nail clipper device.

According to a second example aspect of the present invention, there is provided a method comprising:
using one or more strain gauges comprised in a nail clipper device for sensing transformation of the nail clipper device, the transformation taking place in response to a force being applied to the nail clipper device.

In an example embodiment, the method further comprises using transformations sensed by the one or more strain gauges to determine displacement of clipping edges of the nail clipper device.

In an example embodiment, the method further comprises using transformations sensed by the one or more strain gauges to determine the force applied to the nail clipper device.

In an example embodiment, the method further comprises determining based on readings from the one or more strain gauges at least one of nail thickness, nail strength, changes in nail thickness and changes in nail strength.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 shows a nail clipper device according to an example embodiment;
Fig. 2 is a simplified qualitative diagram illustrating sensed force and displacement in an example nail clipper device;
Fig. 3 shows a nail clipper according to another example embodiment;
Figs. 4A and 4B show schematic top and side views of an example guiding member according to an example embodiment;
Fig. 5 shows a simplified block diagram of a nail clipper device according to an example embodiment;
Figs. 6A and 6B show simplified block diagrams of a system according to an example embodiment;
Figs. 7-9 show flow charts of processes of example embodiments; and
Fig. 10 shows a simplified qualitative diagram illustrating changes in nail characteristics over time.

### DETAILED DESCRIPTON OF THE DRAWINGS

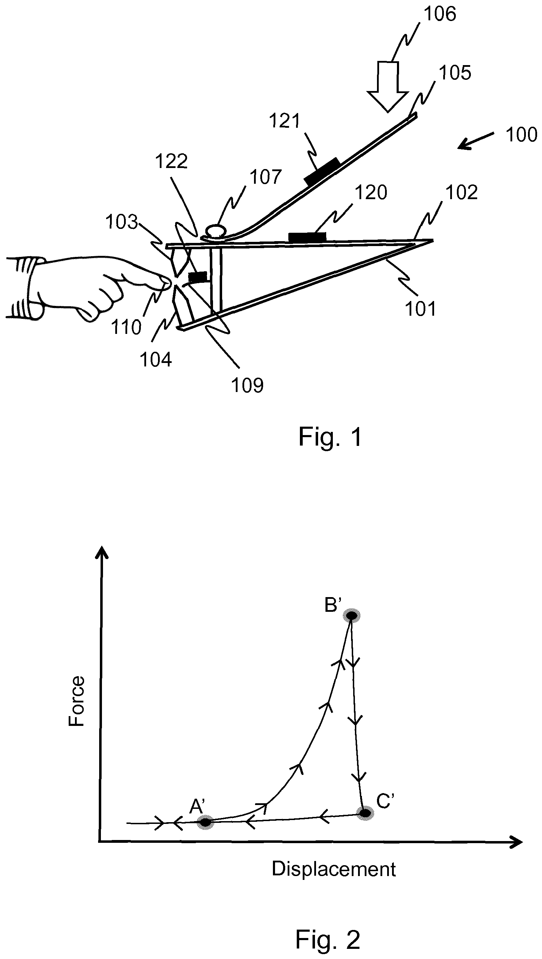

Example embodiments of the present invention and its potential advantages are understood by referring to Figs. 1 through 10 of the drawings. In this document, like reference signs denote like parts or steps.

Persons commonly at risk of calcium or vitamin D deficiency include include those with inadequate sun exposure, limited oral intake (e.g. nutrition), or impaired intestinal absorption. According to Cashman, K. D. (2007), Calcium and vitamin D. In Dietary Supplements and Health: Novartis Foundation Symposium 282 (pp. 123-142), John Wiley & Sons, Ltd. significant proportions of some population groups fail to achieve the recommended calcium intakes in a number of western countries. Calcium is required for normal growth and development as well as maintenance of the skeleton. Vitamin D is also essential for intestinal calcium absorption and plays a central role in maintaining calcium homeostasis and skeletal integrity. In addition, both micronutrients have important roles in non-skeletal-related physiological processes.

Characteristics of human or animal nails or changes thereof may be may be used as an indicator of personal calcium or vitamin D levels. Nail characteristics may be usable an indicator of other health conditions or nutritional deficiencies, too. Nail characteristics being affected e.g. by calcium level is discussed for example in the following publication: Pravina, P., Sayaji, D. and Avinash, M. (2013), Calcium and its Role in Human Body, International Journal of Research in Pharmaceutical and Biomedical Sciences Vol. 4 (2) Apr-Jun 2013.

In various example embodiments of the invention there is provided a new device suited for monitoring nail characteristics of a subject. The subject may be a human or an animal. The monitoring results or changes thereof over time may then be used for analysing health conditions of the subject and providing feedback concerning the health conditions.

In the following, various example embodiments are mainly discussed in connection with monitoring characteristics of fingernails of a human being. The embodiments are however applicable for monitoring characteristics of toenails or nails of animals, too.

Various example embodiments provide an apparatus that comprises comprises nail clipping edges that are configured to move towards each other when the apparatus is actuated. The apparatus is for example a nail clipper device and in the following disclosure a nail clipper device is used as an example of the apparatus. The nail clipper device is actuated by applying a force to the nail clipper device. In an embodiment a first clipping edge of the nail clipper device is configured to move upwards and a second clipping edge of the nail clipper device is configured to move downwards in response to the force. Furthermore it is to be noted that either both nail clipping edges may move, or only one of the nail clipping edges may be configured to move in response to the force. If a nail is placed between the nail clipping edges, a piece of the nail is clipped in response to a sufficient level force actuating the nail clipper device. The nail clipper device may have a compound lever type structure or a plier type structure.

In an embodiment such nail clipper device is furnished with one or more strain gauges configured to sense transformations of the nail clipper device caused by the force. The strain gauges may be configured to sense transformations of the nail clipper device during clipping. Readings from the strain gauges may be used for determining nail characteristics such as nail thickness, nail strength and/or nail length. Nail characteristics and/or changes thereof may then be used as an indicator of certain health conditions.

In general a strain gauge is a device used to measure strain on an object. A strain gauge is a sensor that is attached to the element that is being monitored. The strain in the element is measured by measuring changes in electrical resistance of the sensor. Examples of suitable strain gauge types include: a metal thin film based strain gauge, a nanoparticle based strain gauge, an elastomer strain gauge, a piezoelectric polymer strain gauge, a semiconductor strain gauge, and a fiber-optic strain gauge.

In an embodiment the nail clipper device comprises a first strain gauge configured to sense a transformation that is proportional to displacement of the clipping edges and a second strain gauge configured to sense transformation that is proportional to the force applied to the nail clipper device. The nail clipper device may further comprise a third strain gauge configured to sense transformation that is proportional to length of the free edge of a nail placed between the nail clipping edges (i.e. the piece of nail that is being clipped from the nail). In the following, the example implementations comprise at least two strain gauges, but alternatively only one strain gauge may be used, if there is a need to monitor only thickness, strength or length of the nail. In the following example implementations the two strain gauges are used for monitoring nail thickness and nail strength. In other embodiments, other nail characteristics may be monitored. For example, nail length and nail thickness may be monitored, or nail length and nail strength may be monitored.

Fig. 1 shows a nail clipper 100 according to an example embodiment. embodiment. The nail clipper 100 has a compound lever type structure comprising main arms 101 and 102, nail clipping edges 103 and 104, and a lever arm 105. The main arms 101 and 102 are connected to each other at their first end thereby forming a main arm structure and the nail clipping edges 103 and 104 are respectively located in the second ends of the main arms 102 and 101. A first end of the lever arm 105 is pivotally attached to the main arm structure through a pin 107. In initial position the nail clipping edges 103 and 104 are spaced apart from each other. When a force 106 (depicted by arrow) is applied to a second, free end of the lever arm 105, the nail clipping edges 103 and 104 move towards each other to provide a clipping effect. If nail 110 is placed in the space between the nail clipping edges 103 and 104 in initial position, the actuation of the nail clipper device 100 by the force 106 causes the nail 110 being clipped.

The nail clipper device 100 further comprises strain gauges 120 and 121. The strain gauge 120 is attached to the main arm 102 and the strain gauge 121 is attached to the lever arm 105. In an embodiment the strain gauges 120 and 121 are respectively located in the middle of the main arm 102 and the lever arm 105. Alternatively, other placements may be used. The strain gauges 120 and 121 are configured to sense transformations of the nail clipper caused by actuation of the nail clipper by the force 106. Operation of the strain gauges 120 and 121 is discussed in more detail below e.g. in connection with Fig. 2.

Fig. 1 shows also a spring 109 and a third strain gauge 122 of certain further embodiment. It is to be noted that these are not required in various other embodiments. That is, these are not mandatory in the nail clipper device of Fig. 1. The purpose of the spring 109 and the third strain gauge 122 is to enable monitoring of length of free edge of nail clipping. When a user places the nail 110 between the nail clipping edges 103 and 104, the spring 109 deflects and reading from the third strain gauge 122 can be used for determining the length of the free edge of the nail that is about the be clipped. By making a plurality of such length determinations over time one may monitor nail growth over time.

Fig. 2 is a simplified qualitative diagram illustrating determined force determined force and displacement in an example nail clipper, e.g. the nail clipper 100 of Fig. 1, during a clipping session. X-axis of the diagram gives displacement of the nail clipping edges 103 and 104 from their initial position (i.e. the size of the opening between the nail clipping edges may be deduced from this) and Y-axis of the diagram gives the force applied to the nail clipper to actuate it. It is to be noted that the diagram of Fig. 2 is a simple qualitative example that does not give actual values.

Initially no force is applied. Then the user starts to press the lever arm of the nail clipper and to apply the force 106 in Fig. 1. The lever arm 105 and the main arm 102 of Fig. 1 start to bend in response to the force. The strain gauge 120 senses deflection of the main arm 102. The reading of the strain gauge 120 is proportional to the displacement of the nail clipping edges. The reading of the strain gauge 120 can be considered to be a linear function of the displacement of the nail clipping edges. The strain gauge 121 senses deflection of the lever arm 105. Reading of the strain gauge 121 is proportional to the applied force. Reading of the strain gauge 121 can be considered to be a linear function of the applied force (Young's modulus gives the relation between the strain gauge 121 reading and the applied force).
- In the beginning of the clipping session, the force that is required to press the lever arm is low until the nail clipping edges contact the nail 110; point A' in Fig. 2.
- After point A' the required force increases as the nail clipping edges are in contact with the nail, and the force curves up from point A' to point B'.
- With a large enough force being applied to the lever arm 105, the nail 110 becomes clipped (point B') and the nail clipping edges 103 and 104 contact each other and the force required is reduced; point C' in Fig. 2.
- The difference of readings of the strain gauge 120 between points A' and C' can be used to determine nail thickness (this may require calibrating strain gauge120 with real nail clipping edge displacement).
- The difference of the readings of the strain gauge 121 between points A' and B' can be used to determine the maximum force that can be applied before the nail becomes clipped (nail's maximum tensile strength).

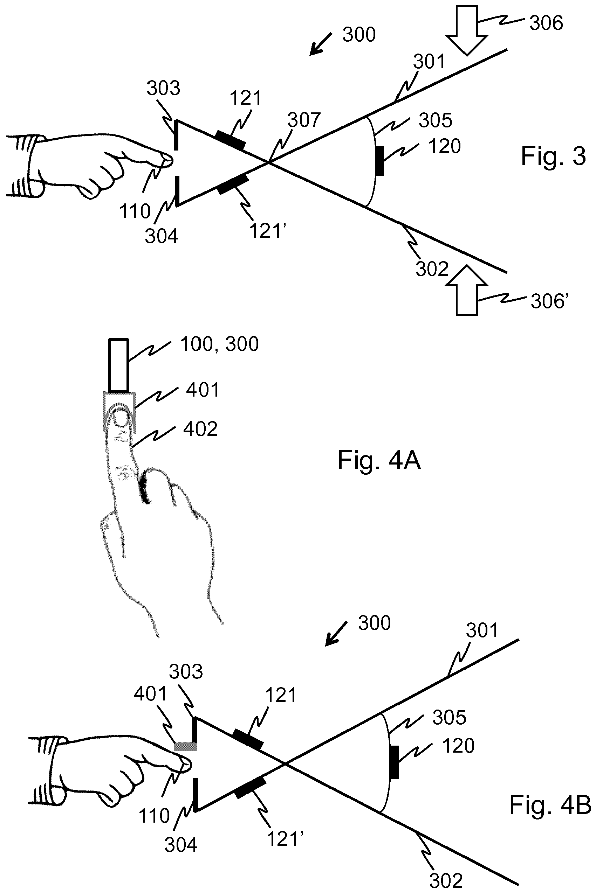

Fig. 3 shows a nail clipper device 300 according to another example embodiment. The nail clipper device 300 has a plier type structure comprising main arms 301 and 302, and nail clipping edges 303 and 304. The main arms 301 and 302 are pivotally connected to each other at point 307. First ends of the main arms 301 and 302 are free and the nail clipping edges 303 and 304 are respectively located in the second ends of the main arms 302 and 301. Additionally the nail clipper 300 comprises a spring 305 that connects the main arms 301 and 302 to each other. In initial position the nail clipping edges 303 and 304 are spaced apart from each other. When forces 306 and 306' (depicted by arrows) are applied to the main arms 301 and 302 at or near their second ends, the nail clipping edges 303 and 304 move towards each other to provide a clipping effect. If a nail 110 is placed between the nail clipping edges 303 and 304 in initial position, the actuation of the nail clipper 300 by the forces 306 and 306' causes the nail 110 being clipped.

The nail clipper device 300 further comprises a strain gauge 120 attached to the spring 305 and strain gauges 121 and 121' attached to the main arms 301 and 302. The strain gauges 120, 121, and 121' are configured to sense transformations of the nail clipper device caused by actuation of the nail clipper device by the forces 306 and 306'.

When a nail is placed between the nail clipping edges 303 and 304 and forces 306 and 306' are being applied, the main arms 301 and 302 and the spring 305 start to bend in response to the forces. The strain gauge 120 senses transformation of the spring 305. Reading of the strain gauge 120 is proportional to the displacement of the clipping edges. The strain gauges 121 and 121' sense transformation of the main arms 301 and 302. Readings of the strain gauges 121 and 121' are proportional to the applied forces.

The strain gauges (e.g. strain gauges 120, 121, 121' and 122 of Figs. 1 and 3) may be attached to the nail clipper structure for example by a suitable adhesive or by some other suitable manner. In an example implementation, the strain gauges are formed as an integral part of the nail clipper structure. Figs. 1 and 3 show the strain gauges only on one side of the arms of the nail clippers, but in practice there may be two strain gauges on opposite sides of the arms. Furthermore, it is to be noted that sizes and locations of the strain gauges in Figs. 1 and 3 are only illustrative and do not necessarily reflect actual sizes or locations. Additionally, it is noted that details disclosed in connection with one figure or one embodiment may be combined to embodiments of other figures.

In order to produce easily repeatable and thereby comparable monitoring events it may be beneficial to ensure that the nail is placed between the clipping edges in the same way every time. An embodiment provides a guiding member for guiding the nail/finger/toe to a desired clipping position. The guiding member may be an accessory device or an integral part of the nail clipper device. Figs. 4A and 4B show schematic top and side views of an example of such guiding member according to an example embodiment. Fig. 4A shows a nail clipper device 100, 300 (e.g. the nail clipper device of Fig. 1 or the nail clipper device of Fig. 3) and a guiding member 401 attached to the nail clipper device 100, 300. The guiding member 401 comprises an opening for receiving a finger 402 and guiding the finger 402 to the nail clipper device in desired position (e.g. in certain angle and/or in certain vertical or horizontal position). Fig. 4B shows an example where such guiding member 401 is attached to a nail clipper device 300 of Fig 3. In the shown example the guiding member is attached to the nail clipping edge 303 of the nail clipper device 300.

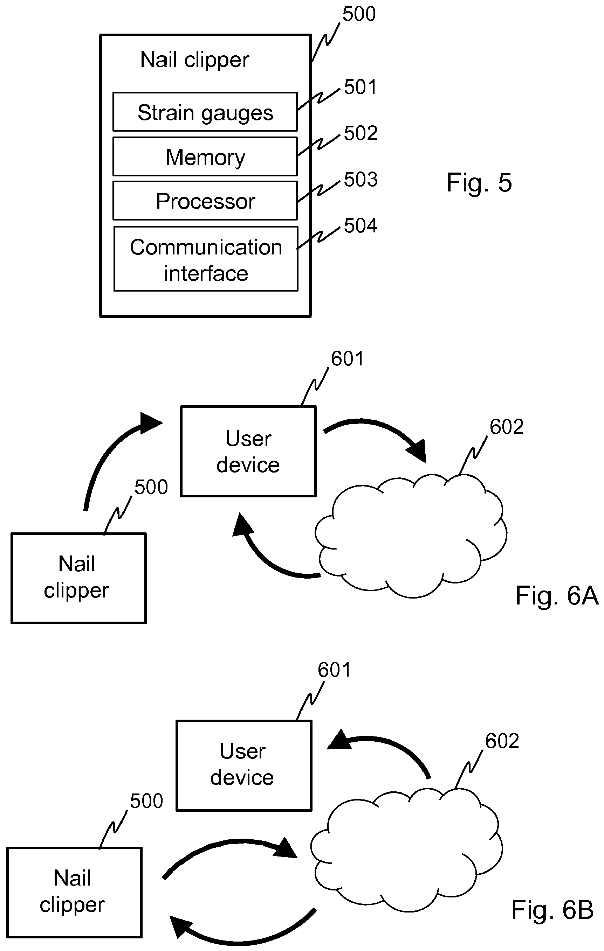

Fig. 5 shows a simplified block diagram of a nail clipper device 500 according to an example embodiment. The nail clipper device 500 comprises strain gauges 501, a memory 502, a processor 503 and a communication interface 504. The memory 502 may be a buffer memory configured for temporary storage or the memory may operate as a longer-term storage. The processor 503 may be for example a microprocessor or some suitable logic component. The communication interface may be for example a Bluetooth interface or some other short-range communication interface, or a NarrowBand loT interface.

The strain gauges 501 are configured to monitor transformations of the nail clipper device. The processor 503 is configured to obtain readings from the strain gauges 501 and to store the readings to the memory 502. In practise the nail clipper device 500 comprises a measurement circuitry (not shown in Fig. 5) configured to measure a property (e.g. resistance) of the strain gauges 501. There is also an A/D converter that converts the measured property of the strain gauges to a digital value that can be stored in the memory 502. Further the processor 503 is configured to control operation of the communication interface 504 and to transmit readings of the strain gauges 501 to external devices through the communication interface 504. Additionally, the processor 503 may be configured to perform processing of the readings of the strain gauges 501. The processor may for example perform pre-processing of the readings prior to transmitting the readings to external devices. In an embodiment the processor may combine some readings e.g. by taking maximum, minimum, average or mean reading of a set of readings. It is to be noted that any processing of the readings in the processor of the nail clipper device is however not mandatory in embodiments of the invention.

In certain embodiments the nail clipper device may further comprise a display or other user interface (not shown in Fig. 5) configured to provide output to the user. The user interface may for example show readings from the strain gauges or monitoring results obtained using the readings from the strain gauges or recommendations or advise derived on the basis of the readings from the strain gauges.

Figs. 6A and 6B show a simplified block diagram of a system according to an example embodiment. The system in both Figs. 6A and 6B comprises a nail clipper device 500, a user device 601 and a cloud service 602. The nail clipper device 500 is furnished with strain gauges configured to sense transformations of the nail clipper device. The user device 601 is an external electronic device such as a smart phone.

In Fig 6A the nail clipper device 500 is configured to transmit readings from the strain gauges to the user device 601 over a communication interface. The communication interface is for example a Bluetooth connection or some other short-range connection. The user device 601 may comprise a user application configured to process the readings transmitted from the nail clipper device 500. The user application may perform analysis of the readings and provide to the user through a user interface of the user device certain monitoring results obtained using the readings from the strain gauges or recommendations or advice derived on the basis of the readings from the strain gauges.

In an embodiment the user device 601 or the user application therein does not perform analysis of the readings from the strain gauges of the nail clipper device 500. Instead the user device 601 may send the readings to an external device such as the cloud service 602 over a suitable communication connection, e.g. over a wireless cellular connection or over a wireless local area connection. The cloud service 602 may comprise for example data acquisition functionality, analytics functionality and decision support functionality. In an embodiment the readings from the strain gauges of the nail clipper device are automatically transferred to the cloud service through the user device. The cloud service analyses the readings and provides results of the analysis back to the user device for being output to the user through a user interface of the user device. The cloud service may provide certain monitoring results obtained using the readings from the strain gauges or recommendations or advise derived on the basis of the readings from the strain gauges.

It is to be noted that the user device may perform some analysis or pre-processing of the reading of the strain gauges or the analysis may be fully performed in the cloud service 602. Likewise some pre-processing may take place already in the nail clipper device. Additionally, it is possible that at least part of the functionality of the user device 601 and the user application therein is implemented in the nail clipper device.

In Fig 6B the nail clipper device 500 is configured to transmit readings from the strain gauges to an external device such as the cloud service 602 over a communication interface. The communication interface is for example a NarrowBand loT connection or some other suitable connection. The cloud service 602 may be configured to process the readings transmitted from the nail clipper device 500. The cloud service 602 may comprise for example data acquisition functionality, analytics functionality and decision support functionality. The cloud service analyses the readings and provides results of the analysis back to the nail clipper device 500 or to the user device 601 for being output to the user through a user interface. The cloud service may provide certain monitoring results obtained using the readings from the strain gauges or recommendations or advise derived on the basis of the readings from the strain gauges.

It is to be noted that the nail clipper device may perform some analysis or pre-processing of the reading of the strain gauges or the analysis may be fully performed in the cloud service 602. Additionally, the user device 601 may comprise a user application configured to perform some post processing of the results of the analysis provided by the cloud service 602. Such post processing may concern for example processing the results for display purposes.

Above mentioned certain monitoring results obtained using the readings from the strain gauges or recommendations or advise derived on the basis of the readings from the strain gauges may include for example analysis of nail thickness, strength and/or length, trends in nail thickness, strength and/or length, estimation of vitamin D or calcium or other vitamin intake and related recommendations (whether the level is e.g. appropriate or too low), guidance on recommended actions (e.g. changes in nutrition).

In an embodiment readings are recorded from several clipping sessions over time and the recorded readings are used for determining changes in nail thickness, nail strength, and/or nail length. The determined changes may then be used for determining changes in vitamin or calcium intake or other health conditions. Individual readings may be used if personal baseline information is available for the analysis.

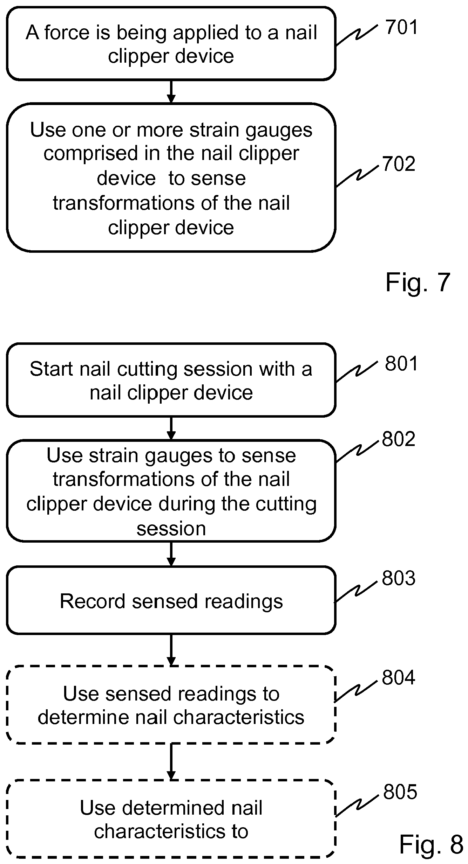

Fig. 7 shows a flow chart of a process of an example embodiment. The process may be implemented for example in the nail clipper device of Figs. 1, 3, 5 or 6A-B. The process comprises the following phases:
701: A force is being applied to the nail clipper device.
702: One or more strain gauges comprised in the nail clipper device are used to sense transformations of the nail clipper device. The sensed transformations may then be processed in the nail clipper device or elsewhere to determine nail characteristics and further to analyse certain health conditions.

Fig. 8 shows a flow chart of a process of an example embodiment. The process may be implemented for example in the nail clipper device of Figs. 1, 3, 5 or 6A-B. Some of the shown process phases may be implemented in the user device or cloud service of Figs. 6A and 6B. The process concerns determining nail characteristics for certain purposes, such as determining vitamin intake or changes thereof. The process comprises the following phases:
801: The process is started. A nail clipping session is started. A nail clipper device according to embodiments of the invention is used for this purpose. The nail clipper device comprises strain gauge(s).
802: The strain gauge(s) are used for sensing transformations of the nail clipper device during the nail clipping session.
803: Sensed readings are recorded. The readings may be stored e.g. in a memory in the nail clipper device. A time stamp may be recorded with each session or with each reading. In order to be able to provide the time stamp, the nail clipper device comprise a clock, or the nail clipper device may have access to a clock via a remote connection. The time stamps may be later used for monitoring nail characteristics over time.
804: The sensed readings are used for determining nail characteristics. If multiple readings are obtained over time, changes in nail characteristics may be determined. It is to be noted that this phase may be implemented in the nail clipper device but that in an alternative implementation this is performed outside the nail clipper device, e.g. in an external user device, such as smart phone, or in a cloud service.

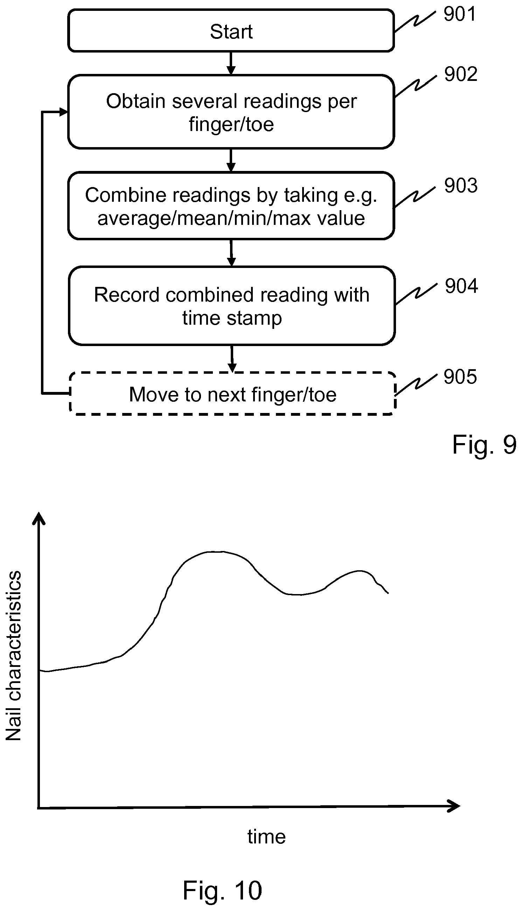

Fig. 9 shows a flow chart of a process of an example embodiment. The process may be implemented for example in nail clipper device of Figs. 1, 3, 5 or 6A-6B. Some of the shown process phases may be implemented in the user device or cloud service of Figs. 6A and 6B. The process concerns determining nail characteristics for certain purposes, such as determining vitamin intake or changes thereof. The process comprises the following phases:
901: The process is started. A nail clipping session is started. A nail clipper device according to embodiments of the invention is used for this purpose. The nail clipper device comprises strain gauge(s) and the strain gauge(s) are used for sensing transformations of the nail clipper device during the nail clipping event.
902: Several readings are obtained per finger or toe. For example only part of a nail is clipped at a time.
903: Set of readings obtained per finger or toe are combined into one reading per finger or toe e.g. by taking average, mean, minimum or maximum over the set of readings.
904: The combined readings are recorded in memory with a time stamp. There may be one time stamp for the session or each combined reading may be provided with an individual time stamp. In order to be able to provide the time stamp, the nail clipper device comprise a clock, or the nail clipper device may have access to a clock via a remote connection. The time stamps may be later used for monitoring nail characteristics over time.
905: The process moves to the next finger or toe and back to phase 802 to obtain several readings for that one.

It is to be noted that phases 903 and 904 may be implemented in the nail clipper device but that in an alternative implementation this is performed outside the nail clipper device, e.g. in an external user device, such as smart phone, or in a cloud service.

Nail clipper device of various embodiments may be configured to give guidance of which nail to clip or there may be a user interface through which the user may indicate which nail is being clipped.

Fig. 10 shows a simplified qualitative diagram illustrating changes in changes in nail characteristics over time. It is to be noted that the diagram of Fig. 10 is a simple qualitative example that does not give actual values. X-axis gives time and Y-axis indicates certain nail characteristic value. Shown hypothetic example plot indicates that nail characteristics may vary over time. For example time of the year or weather conditions or changes in nutrition may affect the readings. For example the user application of the user device 601 of Figs. 6A and 6B may give such diagram as an output.

Without in any way limiting the scope, interpretation, or application of the claims appearing below, a technical effect of one or more of the example embodiments disclosed herein is an easy to use health monitoring arrangement. According to various embodiments the monitoring takes place at the same time with nail clipping event and thereby processing of any samples is not needed. The monitoring may take place automatically during a clipping event without requiring further actions.

Another technical effect of one or more of the example embodiments disclosed herein is a device that enables vitamin D level monitoring without blood samples. Likewise the device may enable also monitoring levels of other vitamins and/or calcium level monitoring.

Another technical effect of one or more of the example embodiments disclosed herein is easy repeatability of the measurement event.

Another technical effect of one or more of the example embodiments disclosed herein is that the arrangement of various embodiments enables personalized coaching (e.g. nutrition recommendations) based on the monitoring results. Personalized coaching may for example instruct to increase calcium or vitamin D intake to enhance personal overall health. Such instructions may be automatically generated on the basis of the monitoring results.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example example embodiments of the invention, these descriptions should not be viewed in a limiting sense. Rather, there are several variations and modifications, which may be made without departing from the scope of the present invention as defined in the appended claims.

## Claims

1. An apparatus comprising:
nail clipping edges configured to move towards each other when a force is applied to the apparatus, and
one or more strain gauges configured to sense transformations of the apparatus caused by said force.

2. The apparatus of claim 1, wherein the one or more strain gauges comprise a first strain gauge configured to sense a transformation that is proportional to displacement of the nail clipping edges.

3. The apparatus of claim 1 or 2, wherein the one or more strain gauges comprise a second strain gauge configured to sense a transformation that is proportional to the force applied to the apparatus.

4. The apparatus of any preceding claim, wherein the one or more strain gauges comprise a third strain gauge configured to sense transformation that is proportional to length of free edge of a nail placed between the nail clipping edges.

5. The apparatus of any preceding claim, further comprising a guiding member configured to guide a nail between the nail clipping edges.

6. The apparatus of any preceding claim, further comprising a memory configured to store readings of the one or more strain gauges.

7. The apparatus of any preceding claim, further comprising a communication interface configured to transmit readings of the one or more strain gauges to an external device.

8. The apparatus of any preceding claim, further comprising a processor configured to obtain a set of readings from the one or more strain gauges and to combine the set of readings into one reading.

9. The apparatus of any preceding claim, further comprising a processor configured to associate readings of the one or more strain gauges with a time stamp.

10. The apparatus of any preceding claim, wherein the one or more strain gauges comprise: a metal thin film based strain gauge, a nanoparticle based strain gauge, an elastomer strain gauge, a piezoelectric polymer strain gauge, a semiconductor strain gauge, or a fiber-optic strain gauge.

11. The apparatus of any preceding claim, wherein the apparatus is a nail clipper device.

12. A method comprising
using one or more strain gauges comprised in a nail clipper device for sensing transformation of the nail clipper device, the transformation taking place in response to a force being applied to the nail clipper device.

13. The method of claim 12, further comprising using transformations sensed by the one or more strain gauges to determine displacement of clipping edges of the nail clipper device.

14. The method of claim 12 or 13, further comprising using transformations sensed by the one or more strain gauges to determine the force applied to the nail clipper device.

15. The method of claim 12, 13, or 14, further comprising determining based on readings from the one or more strain gauges at least one of nail thickness, nail strength, changes in nail thickness and changes in nail strength.
